# EUROPEAN PATENT SPECIFICATION

(11) **EP 1 973 554 B1**
(45) Date of publication and mention of the grant of the patent: **14.01.2015**
(21) Application number: 06839104.4
(22) Date of filing: 07.12.2006
(51) Int. Cl.: A61K 35/14, A61K 31/765

(54) **CARTILAGE REPAIR METHODS**
VERFAHREN ZUR REPARATUR VON KNORPEL
PROCEDES DE REPARATION DE CARTILAGE

(30) Priority: 07.12.2005 US 748027 P
(43) Date of publication of application: 01.10.2008
(73) Proprietor: ISTO TECHNOLOGIES INC., St. Louis, MO 63132 (US)
(72) Inventor: SEYEDIN, Mitchell, S., Monte Sereno, CA 95030 (US); MATAVA, Matthew, St. Louis, MO 63141 (US)
(74) Representative: Smaggasgale, Gillian Helen
(86) International application number: PCT/US2006/046576
(87) International publication number: WO 2007/067637

(56) References cited:
- WO-A-01/35968
- WO-A-96/28539
- US-A1- 2004 033 212
- US-B1- 6 454 811
- US-B2- 6 626 950
- US-B2- 6 689 747
- US-B2- 6 949 252
- UEMATSU K ET AL: "Cartilage regeneration using mesenchymal stem cells and a three-dimensional poly-lactic-glycolic acid (PLGA) scaffold" BIOMATERIALS, ELSEVIER SCIENCE PUBLISHERS BV., BARKING, GB, vol. 26, no. 20, 1 July 2005 (2005-07-01), pages 4273-4279, XP025280497 ISSN: 0142-9612 [retrieved on 2005-07-01]

## Description

### INTRODUCTION

Defects of articular joints are significant sources of pain, discomfort and disability. These defects, such as full-thickness chondral defects, can be associated with diseases such as osteoarthritis, injury and/or degeneration of articular cartilage. Morbidity associated with defects of hyaline cartilage comprised by articular joints are responsible for significant economic, health and social costs.

Current treatments for repair or amelioration of joint problems include microfracture, abrasion and drilling. These interventions involve exposing a joint containing a defect to mesenchymal stem cells. As a result of such interventions, the mesenchymal stem cells can infiltrate the defect, and differentiate into fibrocartilage over time. However, fibrocartilage has a structure and molecular composition distinct from that of hyaline cartilage (Benjamin, M., et al., International Review of Cytology 233: 1-45, 2004). Fibrocartilage generally provides only short-term improvement, typically lasting less than two years. (See, e.g., Hunziker, E.B., Osteoarthritis and Cartilage 10: 432-463, 2001;
Steadman, J.R., et al., Clinical Orthopaedics and Related Research 391 S: S362-S369, 2001; Diduch, D.R., et al.; Stone, K.R., et al.; Fu, F.H., et al.s Amer. J. Sports Medicine 33: 1658-1666, 2005; Minas, T., et al., Orthopedics 20: 525-538, 1997; Gilbert, J.E., Amer. J. Knee Surgery 11 : 42-46, 1998; Gross, A.E., J. Arthroplasty 18: 14-17, 2003.) Alternative treatments are, therefore, needed.
WO96/28539 describes that mesenchymal stem cells in a polymeric carrier implanted into a cartilage and/or bone defect will differentiate to form cartilage and/or bone, as appropriate. The polymeric carriers include porous meshes or sponges formed of synthetic or natural polymers, as well as polymer solutions. A preferred material is a polyglycolic acid mesh.
Uematsu K et al. (Biomaterials, vol 26, no 20, 1 July 2005, p4273-4279) describes a three-dimensional poly-lactic-poly-glycolic scaffold that is said to provide structural support and stimulate repair, when seeded with cultured mesenchymal stem cells and transplanted into rabbit knees. It is suggested that the defects are filled with smooth, shiny white tissue macroscopically and a hyaline-like cartilage histologically after 12 weeks from transplantation.
WO01/35968 describes methods of producing cartilage *in vitro,* methods of promoting chondrocytic differentiation, methods of enhancing chondrogenesis and methods of producing implantable cartilaginous cultures. The methods feature, in particular, the culturing of pleuripotent mesenchymal stem cells. The methods are useful in the repair of cartilage as well as augmentation of cartilage *in vivo.* Also describes are implantable cartilaginous cultures.
US6689747 discloses methods for the treatment and repair of cartilage, including cartilage damaged by injury or cartilagenous disorders, comprising the administration of insulin and/or insulin variants. Optionally, the administration may be in combination with a cartilage agent (e.g. a peptide growth factor, a catabolism antagonist, an anti-inflammatory factor), in an extended- or sustained-release form. The methods may be used in combination with standard surgical techniques. Alternatively, the methods provide for the treatment and repair of cartilage damaged by injury or cartilagenous disorders comprising the administration of chondrocytes previously treated with an effective amount of insulin and/or insulin variant.
US2004/033212 describes a medicament and method of treating a cartilage defect, as well as an implant for treating a cartilage defect, comprising a higher concentration of immature chondroprogenic cells than at the bone-cartilage interface. The implant may include hyaluronic acid.
US6454811 describes composite devices for tissue engineering having a gradient of one or more of the following: materials, macroarchitecture, microarchitecture or mechanical properties, which can be used to select or promote attachment of specific cell types on and in the devices prior to and/or after implantation. The gradient may form a transition zone in the device from a region composed of materials or having properties best suited to one type of tissue to a region composed of materials or having properties suited to a different type of tissue. The gradient may relate to the materials, the macroarchitecture, the microarchitecture, the mechanical properties of the device, or several of these together.
US6626950 describes a prosthetic implant having a tissue scaffold and a fixation device with a scaffold support and an anchoring post. The anchoring post extends from a surface of the scaffold support at a selected angle with the scaffold support embedded within the scaffold. The scaffold has a porous ceramic phase and a porous polymer phase. The polymer is foamed while in solution thereby creating an interphase junction of interlocked porous materials and embedding the scaffold support portion of the fixation device. The scaffold may be infused or coated with a variety of bioactive materials to induce ingrowth or to release a medicament. US6949252 discloses compositions and methods for preparing tissues or tissue constructs. The construction of a multi-layered biological structure (i.e. system) that includes a cellular support matrix seeded with living cells derived from a native tissue is disclosed. Also disclosed are tissue culture protocols to promote the *in vitro* growth of tissues and tissue constructs.
may be infused or coated with a variety of bioactive materials to include ingrowth or to release a medicament.
US6949252 describes compositions and methods for preparing tissues or tissue constructs. The construction of a multi-layer biological structure (Le. system) that includes a cellular support matrix seeded with living cells derived from a native tissue is disclosed. Also disclosed are tissue culture protocols to promote the *in vitro* growth of tissues and tissue constructs.

In view of the need for therapeutic interventions to treat chondral defects, the present inventors disclose herein a membrane for use in repairing a hyaline cartilage defect in a joint in a mammal such as a human patient in need of treatment. Thus according to the present invention there is provided a membrane in combination with autologous mesenchymal stem cells, said membrane comprising a polyester selected from the group consisting of polylactic acid (PLA), polyglycolic acid (PGA), and a co-polymer comprising polylactic acid and polyglycolic acid (PLGA), the polyester being entangled with hyaluronic acid, for use in a method for repairing a hyaline cartilage defect in a mammalian joint. This can result in deposition of hyaline cartilage instead of fibrocartilage at a defect site, and thereby provide long-term improvement, including complete repair, of a joint defect.

In various configurations of the present teachings, the mesenchymal stem cells can be bone mesenchymal stem cells autologous to the mammal, and can originate in a bone marrow cavity underlying the joint in need of repair. The joint in need of repair can comprise a full-thickness chondral defect. The joint can be any articular joint comprising hyaline cartilage, such as, without limitation, a knee joint.

At least one aperture can be introduced into the bone underlying the joint. The at least one aperture can be sufficiently large to make possible migration of mesenchymal stem cells from a marrow cavity of the bone to the joint, and can thereby make possible establishment of contact between the mesenchymal stem cells and the joint. Introduction of an aperture into a joint can be achieved by any method known to skilled artisans, such as, for example, abrasion, microfracture, or drilling of the bone. When abrasion is used, the abrading can comprise performing abrasion arthroplasty.

In various aspects of the present teachings, a membrane comprising a polyester entangled with a polysaccharide can have a thickness of at least about 0.5 mm up to about 3 mm. A membrane can be applied to a joint prior to aperture introduction, or aperture introduction can precede membrane application.

Membranes of the present teachings include a polyester entangled with a polysaccharide. The polyester which comprises a membrane is composed of polylactic acid, polyglycolic acid, or a co-polymer comprising polylactic acid and polyglycolic acid. In various aspects, the polylactic acid and polyglycolic acid can be in a weight ratio of from about 5: 1 to about 2:1, such as a weight ratio of about 3:1.

The membranes of the present teachings further include a polysaccharide. The polysaccharide is hyaluronic acid. In various aspects, the polyester and polysaccharide can be in a weight ratio of from 99:1 to 1:99, and in certain configurations, the weight ratio of polyester to polysaccharide can be from about 9:1 to about 1:9. In various configurations, a polyester can be entangled with a polysaccharide using methods set forth in US 2006/008530.

The membrane as described herein can be secured to a joint in need of repair. The membrane can be secured to a joint using at least one fastener. A fastener of these configurations can be any fastener known to skilled artisans, such as, without limitation, a biocompatible glue, a suture, a tissue weld, a dart, a staple, a screw, and a tack. In certain aspects, a biocompatible glue can be a fibrin glue.

In certain embodiments of the present teachings, a membrane described herein can further comprise at least one growth factor. Examples of a growth factor which can be comprised by a membrane include, without limitation, TGF-[beta], a bone morphogenetic protein, a growth differentiation factor, ADMP-1, a fibroblast growth factor, a hedgehog protein, an insulin-like growth factor, a platelet-derived growth factor, an interleukin, a colony-stimulating factor, and an activin. In addition, in some embodiments, a membrane can further comprise at least one collagen.

The membrane of the present invention may be used for repairing a full-thickness chondral defect in a joint of a patient in need of treatment. These methods comprise both microfracturing bone underlying the joint, and applying to the joint a membrane comprising a polyester co-polymer comprising polylactic acid and polyglycolic acid in a weight ratio of from about 5:1 to about 2:1, wherein the polyester is entangled with hyaluronic acid. A membrane of these configurations can have a thickness of at least about 0.5 mm up to about 3 mm. These methods can further comprise anchoring the membrane to the joint.

In addition, membrane of the present invention may be used in methods for repair of a full-thickness chondral defect in a joint of a patient in need of treatment. These methods comprise introducing at least one aperture through bone underlying the joint, wherein the at least one aperture makes possible migration of mesenchymal stem cells comprised by a marrow cavity of the bone to the joint, and applying to the joint a membrane comprising hyaluronic acid entangled with a polyester co-polymer comprising polylactic acid and polyglycolic acid in a weight ratio of from about 5:1 to about 2:1, wherein the membrane has a thickness of at least about 0.5 mm up to about 3 mm. In some configurations, the methods further include securing the membrane to the joint.

### DETAILED DESCRIPTION

The present inventors have devised a membrane for use in repairing hyaline cartilage comprised by a joint. The membrane may be used in a method which entails a) infiltrating a joint in need of repair with autologous mesenchymal stem cells and b) applying to the joint the membrane comprising a polyester selected from the group consisting of polylactic acid (PLA), polyglycolic acid (PGA), and a co-polymer comprising polylactic acid and polyglycolic acid entangled with hyaluronic acid. As used herein, the term "mesenchymal stem cells" refers to pluripotent cells which originate within juvenile or adult mesenchymal tissue. Accordingly, for example, autologous mesenchymal stem cells can be autologous bone mesenchymal stem cells, i.e., autologous mesenchymal stem cells which originate within the marrow cavity of a bone.

The membranes of the present invention includes matrices for supporting the repair of a tissue. The matrix comprises a polyester entangled with a hyaluronic acid. In some configurations, a matrix can further comprise at least one growth factor, which can be a TGF-ß, a bone morphogenetic protein, a growth differentiation factor, ADMP-1, a fibroblast growth factor, a hedgehog protein, an insulin-like growth factor, a platelet-derived growth factor, an interleukin, a colony-stimulating factor, and/or an activin. In addition, the matrix of these may further comprise a collagen.

The hyaluronic acid can be both entangled with the polyester, and can also be cross-linked. In some configurations of a matrix comprising cross-linked hyaluronic acid, the hyaluronic acid can be an oxidized polysaccharide. In some alternative configurations, the polysaccharide can be cross-linked via a cross-linking agent. In addition, in various configurations, a crosslinked matrix can include, not only a cross-linked hyaluronic acid and a polyester, but also a growth factor and/or a collagen.

Methods of preparing a matrix of the present teachings comprise entangling, in a mixture, a polyester and a hyaluronic acid. A method of preparing a matrix can further comprise cross-linking a hyaluronic acid. Cross-linking can include oxidizing the hyaluronic acid, and/or contacting the hyaluronic acid with a cross-linking agent. The oxidizing and/or the contacting of the hyaluronic acid with a cross-linking agent can be effected either before or after entangling the hyaluronic acid with the polyester. In addition, a method of preparing a matrix can further include adding to a mixture at least one growth factor and/or a collagen.

The matrix described herein may be implanted in a mammal, such as a human, at a site in need of tissue growth. Promoting tissue growth can include conducting tissue growth, and/or inducing tissue growth. The tissue can be bone, cartilage, soft tissue, or a combination thereof.

The present inventors have devised matrices for supporting repair of a tissue. The inventors have also devised methods for preparing the matrices, and use of the matrices for supporting tissue repair. An entangled polyester-hyaluronic acid matrix of the present invention may be used alone to conduct the growth of tissue, in combination with at least one growth factor to induce the growth of tissue, in combination with cells to induce the growth of tissues, and/or in combination with a collagen or fibrin. "Entanglement" and related terms, as used herein, refers to a state of polymers in melts or concentrated solutions above the overlap concentration, in which polymers interpenetrate one another and motion of the molecules is restricted to movement along a 'virtual tube' which surrounds each molecule.

Accordingly, a matrix of the present teachings comprises a polyester entangled with hyaluronic acid. A polyester comprised by a matrix is polylactic acid (PLA), polyglycolic acid(PGA), or a copolymer comprising PLA and PGA (also referred to as poly(lactide-co-glycolide, PLA-PGA, or PLGA). A polyester such as a PLGA co-polymer can be a biodegradable co-polymer. In some configurations, a PLGA co-polymer comprised by a matrix can comprise PLA and PLG in a weight ratio of about 5:1 to about 2:1, and, in certain aspects, the PLA:PLG ration can be about 3:1 by weight. A PLA-PLG co-polymer can be, for example, a polyester such as a PLGA co-polymer described in Hollinger, J. Biomed. Mater. Res. 17: 71-82,1983.

In some configurations of a matrix, the hyaluronic acid can be a cross- linked hyaluronic acid. The cross-linkage can include any type of cross-linkage known to skilled artisans, for example as disclosed in references such as Laurent, T. C, Acta Chem. Scand. 18: 274-275, 1964; Kuo, J.-W Bioconjugate Chem. 2; 232-241, 1991; Mason, M., Biomaterials 21: 31-36, 2000; or Zhao, X.B., J. Mater. Sci. Mater. Med. 13: 11-16, 2002, and can include an aldehyde cross-linking agent such as formaldehyde or glutaraldehyde, a homobifunctional cross-linking agent or a heterobifunctional cross-linking agent such as a hyaluronic acid-reactive cross-linking agent. In various aspects, a cross-linkage can comprise an oxidized hyaluronic acid, such as a periodate-oxidized hyaluronic acid. In some configurations, a cross-linkage can comprise a covalent attachment between the hyaluronic acid and the polyester, or between the hyaluronic acid and any other matrix component described herein.

In a matrix of the present teachings, the weight ratio of polyester to hyaluronic acid can be between 99:1 to 1:99. In some aspects, the weight ratio of the polyester to the hyaluronic acid can be from about 9:1 to about 1:9.

In some configurations, a matrix of the present teachings can comprise, in addition to a polyester and hyaluronic acid, at least one growth factor. A growth factor which can be comprised by a matrix can be, in non-limiting example, a member of the TGF-ß superfamily, such as TGF-ß1, TGF-ß2, TGF-ß3, or a bone morphogenetic protein (BMP); a growth differentiation factor; ADMP-1; a fibroblast growth factor (FGF) such as acidic FGF or basic FGF; a member of the hedgehog family of proteins, such as indian hedgehog, sonic hedgehog, or desert hedgehog; a platelet-derived growth factor, an interleukin; a colony- stimulating factor; an activin; a member of the insulin-like growth factor (IGF) family, such as IGF-I or IGF-II; a member of the platelet-derived growth factor (PDGF) family, such as PDGF-AP, PDGF-BB and PDGF-AA; a member of the interleukin (IL) family, such as IL-1, IL-2, IL-3, IL-4, IL-5 or IL-6; or a member of the colony-stimulating factor (CSF) family, such as CSF-I , G-CSF, and GM-CSF. A growth factor comprised by a matrix can be a growth factor obtained from a tissue source, or can be a recombinant growth factor produced in vitro, in a cell culture, or in a microorganism using standard molecular biology techniques. In some aspects, a growth factor can be a bone morphogenetic protein, such as, in non- limiting example, BMP-1, BMP-2, BMP-3, BMP-4, BMP-5, or BMP-6. In addition, a matrix can also include at least one collagen, such as, in non-limiting example, type I collagen, type IX collagen, type X collagen, or type XI collagen.

The present inventors have also developed methods for preparing the matrices described herein. The methods described herein utilize laboratory techniques well known to skilled artisans, and guidance can be found in laboratory manuals such as Sambrook, J., et al., Molecular Cloning: A Laboratory Manual, 3rd ed. Cold Spring Harbor Laboratory Press, Cold Spring Harbor, NY, 2001; Spector, D. L. et al., Cells: A Laboratory Manual, Cold Spring Harbor Laboratory Press, Cold Spring Harbor, NY, 1998; and Harlow, E., Using Antibodies: A Laboratory Manual, Cold Spring Harbor Laboratory Press. Cold Spring Harbor, MY, 1999, and textbooks such as Hedrickson et al., Organic Chemistry 3rd edition, McGraw Hill, New York, 1970.

Accordingly, methods of the present teachings comprise forming a mixture comprising a polyester and a hyaluronic acid, and entangling the polyester and the hyaluronic acid in the mixture. Entangling the hyaluronic acid with a polyester can be effected by any method known to those of skill in the art, such as, in non-limiting example, the method described in Example 1 below.

In making a matrix, the hyaluronic acid is entangled with a polyester comprising polylacticacid, polyglycolic acid, or a co-polymer comprising polylactic acid and poiyglycolic acid. When a polyester is a co-polymer comprising PLA and PGA5 the component polymer acids can be in a weight ratio of about 5:1 to about 2:1, such as about 3:1. A co-polymer can be obtained from a commercial supplier, or can be prepared according to well-known techniques, as described in references such as, in non-limiting example, Fukuzaki, Biomaterials 11: 441-446,1990 and Jalil, J. Microencapsulation 7: 297-325, 1990.

In various aspects, a method for forming a matrix can further comprise oxidizing the hyaluronic acid. The oxidation can utilize any method for oxidizing a hyaluronic acid known to skilled artisans, such as, for example periodate oxidation. Oxidizing hyaluronic acid can comprise oxidizing sugar rings on the hyaluronic acid, and can be effected either before or after entangling the hyaluronic acid with a polyester.

Preparing a matrix can also comprise, in some embodiments, covalently cross-linking the hyaluronic acid component of a matrix. The cross-linking of hyaluronic acid can be effected either before or after forming a mixture with a polyester, or entangling the polyester with hyaluronic acid. In some configurations, cross-linking can be effected using an oxidized hyaluronic acid. In addition, in some aspects, cross-linking can be effected by contacting hyaluronic acid with a chemical cross-linker, such as, in non- limiting example, an aldehyde cross-linking agent such as formaldehyde or glutaraldehyde, a homobifunctional cross-linking agent or a heterobifunctional cross-linking agent such as a polysaccharide-reactive cross-linking agent supplied commercially by sources such as Pierce Biotechnology Inc. (Rockford IL) or Molecular Probes/Invitrogen Corporation, Carlsbad, CA.

Preparation of a matrix can comprise forming a mixture wherein the polyester and the hyaluronic acid are combined in a mixture in a weight ratio ranging from about 99:1 to about 1:99; methods of these embodiments can include adding the polyester and the hyaluronic acid in a weight ratio of from about 9:1 to about 1:9. A skilled artisan can, in non-limiting example, determine by routine experimentation an optimal ratio of polyester to hyaluronic acid with respect to physical, chemical, or biological properties of a resulting entangled matrix, such as, in non-limiting example, adhesiveness towards cells, resorption characteristics, stability, flexibility, strength, biocompatibility, and adsorptiveness for macromolecules such as serum proteins or extracellular matrix components. The macromolecular components of a mixture can be entangled by methods well-known to skilled artisans, which can include, in some aspects, freezing and lyophilizing a mixture comprising a polyester and a hyaluronic acid, or wet laying and air drying the mixture.

Forming a matrix of the present teachings can further comprise adding to a mixture comprising a polyester and a hyaluronic acid, at least one growth factor, such as those listed above, and in particular, a bone morphogenetic protein (BMP). The amount and species of a growth factor to add to a mixture can be determined by a skilled artisan by routine experimentation, and can be varied according to the intended use of a matrix. In non-limiting example, a bone morphogenetic protein can be added to a mixture comprising a polyester and a hyaluronic acid to form a matrix which can be used to stimulate bone growth. Forming a matrix can also comprise adding a collagen to a mixture. The collagen can be any type of collagen such as those listed above.

The matrix of the present invention may be used in methods to promote tissue growth in a mammal. There methods comprise implanting in the mammal, at a site in need of tissue growth the matrix of the present invention.
The matrix may further comprise at least one growth factor and/or at least one collagen. In various configurations, a tissue can be bone tissue, cartilage tissue, a soft tissue, or a combination thereof. Accordingly, a mammalian recipient of a matrix of the present teachings can be a human patient in need of treatment, such as, in non-limiting example, an individual having a degenerative disease of bone or cartilage, or an individual in need of joint repair following a traumatic injury. A skilled artisan such as a surgeon can implant a matrix at a site within the body of the patient. The implanted matrix can accelerate or promote the healing of adjacent tissue.

The matrix of the present invention may be used in the manufacturer of a medicament for promoting tissue growth. The method of manufacturing the medicament may be the described method of forming the matrix.

The described methods of joint repair can be applied to any body joint comprising hyaline cartilage, such as, but not limited to, a joint of a knee, an elbow, an ankle, a shoulder, a jaw or a wrist. Furthermore, the membrane can be used with both humans and animals having joint defects, including, without limitation, a mammal such as companion animal or farm animal (e.g., a cat, a dog, a sheep, a cow, a goat, a pig, or a horse). Defects which can be treated can be any form of joint defect involving loss of or damage to hyaline cartilage, such as, but not limited to, a full-thickness defect, a partial thickness defect, an age-related degenerative disease defect such as osteoarthritis, a congenital defect, or an injury resulting from trauma.

Treatment of a joint defect using the membrane disclosed herein can effect repair of a cartilage defect in which new cartilage that deposits following intervention is hyaline cartilage rather than fibrocartilage. In use, the joint can be contacted with autologous mesenchymal stem cells comprised by bone, the joint may then have applied thereto the membrane of the present invention. Most conveniently, such mesenchymal stem cells can be autologous mesenchymal stem cells originating in the bone underlying the damaged joint, although mesenchymal stem cells from other bones can be used as well. Contact between the damaged joint and autologous mesenchymal stem cells from the underlying bone can be effected most readily by introducing one or more apertures into the bone underlying the defective joint. Such apertures need be at least large enough to allow passage of the mesenchymal stem cells from the bone mesenchyme to the joint. Several well-established procedures can be used to form such passages, such as, without limitation, abrasion (such as abrasion arthroplasty), microfracture, and drilling of the bone. These and other treatment procedures are well known to skilled artisans, and are described in references such as Steadman, J.R. et al., Clinical Orthopaedics and Related Research 391 S: S362-S369, 2001, Rodrigo J.J., et al., Osteoarticular injuries of the knee. pp. 2077-2082, In: Chapman, M.W. (ed): Operative Orthopaedics, Vol. 3, 2nd Ed. Lippincott, Philadelphia, PA, 1993; Tippet J.W., Articular cartilage drilling and osteotomy in osteoarthritis of the knee, pp. 325-339, in: McGinty, J.B. (ed): Operative Arthroscopy. Raven Press, New York, NY, 1991; Vangsness, C.T., et al., Amer. J. Orthop. 33 (2 Suppl): 29-34, 2004; Textbook of Arthroscopy, Miller, M.D. et al., ed. Saunders, 2004; The Adult Knee, Callaghan, JJ. et al., ed., Lippincott Williams & Wilkins, 2003; Operative Treatment of Elbow Injuries, Baker, C.L., et al., ed., Springer, 2002; Osteoarthritis : Fundamentals and Strategies for Joint-preserving Treatment, Grifka, J.J., et al., ed., Springer, 2000; Reconstructive Surgery of the Joints, Morrey, B.F., et al., ed., Churchill Livingstone, 1996; Operative Arthroscopy, McGinty, J.B., et al., ed., Lippincott-Raven, 1996; The Knee, Scott, W.N., ed., Mosby-Year Book, 1994; Surgical Repair and Reconstruction in Rheumatoid Disease, Benjamin, A., et al., Spring-Verlag, 1993; The Knee: Form, Function, Pathology, and Treatment; Larson, R.L., et al., ed., W.B. Saunders, 1993; and O'Connor's Textbook of Arthroscopic Surgery, Shahriaree, H., ed., J.B. Lippincott, 1992. Without being limited by theory, it is believed that following introduction of passages into the bone, mesenchymal stem cells can migrate out from the bone marrow cavity through the passages, and populate the joint. Exposure of the mesenchymal stem cells to the local environment of the joint leads to differentiation of the stem cells into cartilage- forming chondrocytes. In the presence of a membrane comprising a polyester and hyaluronic acid, the chondrocytes produce hyaline cartilage rather than fibrocartilage. The introduction of the cells under these conditions can thereby restore the cartilage of a defective joint to a state more closely resembling that of the joint pre-injury.

The membrane of the present invention can have a thickness of at least about 0.5 mm up to about 3 mm. These membranes can be prepared by methods set forth herein. As described therein, a polyester of a membrane comprises polylactic acid, polyglycolic acid, or a co-polymer of polylactic acid and polyglycolic acid (a "PLA/PLG polymer"). In some aspects, the weight ratio of polylactic acid to polyglycolic acid in a PLA/PLG polymer can be from about 5: 1 to about 2: 1, for example about 3:1. As used herein, the term "hyaluronic acid" can refer to the free acid form of hyaluronic acid, a salt of hyaluronic acid such as sodium hyaluronate, or a combination thereof. In some configurations, the hyaluronic acid can be a commercially available hyaluronic acid, such as a hyaluronic acid distributed by Lifecore Biomedical, Inc, Chaska, MN and can have a weight average molecular weight of from about 100,000 to about 2,000,000 Daltons. In non-limiting example, the hyaluronic acid can be sodium hyaluronate having an average molecular weight of about 1,700,000. In various aspects, the weight ratio of the polyester to the polysaccharide can be from 99:1 to 1:99! In some configurations, the ratio of the polyester to the polysaccharide can be from about 9:1 to about 1:9.

A skilled artisan, such as, for example, an orthopaedic surgeon, can shape a membrane into a shape appropriate for a particular joint defect. The appropriate shape can be determined according to principles well known to skilled artisans, for example, by following guidelines for medical treatment of chondral defects set forth in standard texts such as those listed above.

The membrane may be secured to the joint. Securing the membrane to the joint can be part of the surgical intervention in the treatment of a patient. Accordingly, a skilled artisan such as an orthopaedic surgeon can immobilize a membrane at the site of defect in a patient, using at least one fastener, and thereby retain the membrane at the site. Such retention of the membrane can promote the formation of hyaline cartilage by chondrocytes differentiated from mesenchymal stem cells. Examples of a fastener that can be used include, without limitation, a biocompatible glue, a suture, a tissue weld, a dart, a staple, a screw, a tack, and a combination thereof. A biocompatible glue can be a fibrin glue, such as a fibrin sealant. A non-limiting example of a biocompatible glue that can be used with the present teachings is a fibrin sealant manufactured by Oesterreichisches Institut Fuer Haemoderivate G.M.B.H. in Vienna, Austria and distributed by Baxter Healthcare Corporation, Glendale, CA under the brand name TISSEEL<(R)> VH. Non-limiting examples of other fasteners which can be used instead of, or in addition, a biocompatible glue include sutures, tissue welds such as described in Helmsworth, T. F., et al., Laser Surgery Medicine 10: 576-583, 1990, staples, darts, pins and tacks. In some aspects, a fastener can comprise a biocompatible material such as, without limitation, a PLA/PLG polymer.

In some aspects, a membrane of the present teachings can further comprise one or more growth factors. Without being limited by theory, it is believed that certain growth factors can promote formation of hyaline cartilage by promoting differentiation of mesenchymal stem cells into hyaline cartilage-forming chondrocytes, and can thereby speed healing. Non-limiting examples of growth factors which can be incorporated into a membrane of the present teachings include a fibroblast growth factor such as basic fibroblast growth factor (bFGF), a transforming growth factor such as transforming growth factor-[beta] (TGF-[beta]), a bone morphogenetic protein (BMP) such as BMP-2, ADMP-I3 a hedgehog protein, an insulin-like growth factor, a platelet-derived growth factor, an interleukin, a colony-stimulating factor, and an activin. Furthermore, in some configurations, a membrane can comprise, in addition to or instead of a growth factor, a collagen such as type I collagen or type II collagen. Amounts of a growth factor or collagen to be incorporated into a membrane can be determined by a skilled artisan without undue experimentation.

The matrix of the present invention may be used in methods for repairing a full-thickness chondral defect in a joint of a patient in need of treatment. These methods comprise both microfracturing bone underlying the joint, and applying to the joint a membrane comprising a polyester co-polymer comprising polylactic acid and polyglycolic acid in a weight ratio of from about 5:1 to about 2:1, wherein the polyester is entangled with hyaluronic acid. A membrane of these configurations can have a thickness of at least about 0.5 mm up to about 3 mm. These methods can further comprise anchoring the membrane to the joint. A joint of these configurations can be any joint comprising articular cartilage, such as a joint of a long bone, for example a knee joint comprising articular cartilage of a femur, In these configurations, the micro fracturing can precede the application of a membrane, or vice versa.

Furthermore, the matrix can be used in methods for repair of a full-thickness chondral defect in a joint of a patient in need of treatment. These methods comprise a) introducing at least one aperture through bone underlying the joint, wherein the at least one aperture makes possible migration of mesenchymal stem cells comprised by a marrow cavity of the bone to the joint, and b) applying to the joint a membrane comprising hyaluronic acid entangled with a polyester co-polymer comprising polylactic acid and polyglycolic acid in a weight ratio of from about 5:1 to about 2:1, wherein the membrane has a thickness of at least about 0.5 mm up to about 3 mm. The methods can further comprise securing the membrane to the joint, using attachments methods and devices well known to skilled artisans.

In these methods, introduction of at least one aperture can precede application of a membrane to the joint, or application of a membrane to the joint can precede the introduction of at least one aperture. Furthermore, in these methods, a membrane can be secured to a joint by applying at least one fastener to the membrane and to the joint. Non- limiting examples of a fastener include a biocompatible glue such as a fibrin glue, a suture, a tissue weld, a dart, a staple, a screw, and a tack. A fastener of these methods can be made of a bioabsorbable material such as a polyester, or of non-absorbable material such as a biocompatible metal. Accordingly, in non-limiting example, a fastener can be an absorbable suture which passes through both the membrane and a joint, and thereby secures apposition of the membrane to the joint. Furthermore, in non-limiting example, the attaching can comprise glueing the membrane to the joint.

In various aspects, the methods described herein can be applied to any mammal, including a human patient in need of treatment. In addition to a human, the membrane can be applied to any mammal, such as, in non-limiting example a companion animal such as a cat or dog, or a farm animal such as a horse, a bovine, a goat, a pig or a sheep.

The following examples are illustrative, and are not intended to limit the scope of the claims. The description of a composition or a method in an example does not imply that a described composition has, or has not been produced, or that the described method has been performed, regardless of verb tense used.

The methods described herein utilize laboratory techniques well known to skilled artisans, and guidance can be found in laboratory manuals such as Sambrook, J., et al., Molecular Cloning: A Laboratory Manual, 3rd ed. Cold Spring Harbor Laboratory Press, Cold Spring Harbor, NY. 2001; Spector, D. L. et al., Cells: A Laboratory Manual, Cold Spring Harbor Laboratory Press, Cold Spring Harbor, NY. 1998; and Harlow, E., Using Antibodies: A Laboratory Manual, Cold Spring Harbor Laboratory Press, Cold Spring Harbor, NY. 1999.

### EXAMPLES

### Example 1

This example illustrates a method of constructing an entangled matrix comprising a polyester and hyaluronic acid.

In this example, poly(lactide-co-glycolide) having molecular weight of 1.5x 0⁵ is dissolved in dichloromethane (125 mg/ml) and with Hyaluronate (HA) of molecular weight of about 1.3 x 10⁶ Dalton is dissolved in water (15 mg/ml). The two polymer solutions, 2 parts PLGA, and 1 part HA, are mixed with 1 part Milli Q water by vortexing at high speed for about 5 minutes. The emulsified mixture is immediately poured into a mold pre-cooled at -70 C in a bath containing dry ice in isopropyl alcohol. After freezing, the mold and its contents are transferred into a second container that is loaded with dry ice and connected to vacuum line. Organic solvent is removed by this process at the temperature between -70 C to -40 C, leaving HA in wet-ice phase. Water is then removed by raising the temperature to -10 C under vacuum.

### Example 2

This example illustrates treatment of a knee injury.

In this example, an athletic patient presents with a traumatic knee injury to an orthopedic surgeon. A diagnosis is made of damaged articular cartilage of the femoral condyle. The surgeon performs a microfracture procedure on the patient's femoral condyle, creating channels through the bone underlying the hyaline cartilage. The surgeon selects a membrane comprising polyester entangled with hyaluronic acid, and shaped to follow the contours of the condyle. The polyester is a copolymer of lactic acid and polyglycolic acid in a weight ratio of 3:1 and has a weight average molecular weight of 100,000. The hyaluronic acid has an average molecular weight of 1,700,000. The weight ratio of polyester to hyaluronic acid is 9:1, and the membrane and has a thickness of 3 mm. The surgeon coats one side of the membrane with TISSEEL^{®} VH fibrin sealant. She then applies the membrane to the damaged femoral condyle using gentle pressure. The patient is instructed to keep pressure off the knee for a period of weeks. The condyle is repaired with new hyaline cartilage by six months after the surgical intervention.

### Example 3

This example illustrates treatment of osteoarthritis.

In this example, a patient with osteoarthritis presents with a full-thickness chondral defect in an elbow joint. The patient is operated upon by a surgeon, who performs a microfracture procedure on the humerus underlying the joint. A membrane comprising a polyester entangled with hyaluronic acid, and shaped to follow the contours of the condyle of the humerus, is then positioned by the surgeon upon the condyle. The polyester is a copolymer of lactic acid and polyglycolic acid in a weight ratio of 4:1 and has a weight average molecular weight of 100,000. The hyaluronic acid has a weight average molecular weight of 1,700,000. The weight ratio of polyester to hyaluronic acid is 8:1. The membrane has a thickness of 1 mm. The surgeon secures the membrane in place with a series of screws made of a resorbable PLA/PLG polymer. Following surgery, new hyaline cartilage deposits along the condyle over a six month period. The new cartilage is anatomically indistinguishable from normal hyaline cartilage.

### Example 4

In this example, a middle age male presents with a traumatic dislocation of the shoulder. A diagnosis is made of disruption of the articular cartilage covering the head of the humerus at its articulation with the glenoid socket of the scapula. The patient is operated upon by a surgeon, who performs a microfracture, procedure on the head of the humerus. A membrane comprising a polyester entangled with hyaluronic acid, and shaped to approximate the contours of the humeral head, is then positioned by the surgeon upon the humeral head. The polyester is a copolymer of lactic acid and polyglycolic acid in a weight ratio of 3:1 and has a weight average molecular weight of 200,000. The hyaluronic acid has a weight average molecular weight of 1,700,000. The weight ratio of polyester to hyaluronic acid is 9:1. The membrane has a thickness of 1 mm. The surgeon secures the membrane in place with a series of resorbable pins. Following surgery, new hyaline cartilage deposits along the condyle over a period of six months. The new cartilage is anatomically indistinguishable from normal hyaline cartilage.

Any discussion of references cited herein is intended merely to summarize the assertions made by their authors and no admission is made that any reference or portion thereof constitutes relevant prior art. Applicants reserve the right to challenge the accuracy and pertinency of the cited references.

## Claims

1. A membrane in combination with autologous mesenchymal stem cells, said membrane comprising a polyester selected from the group consisting of polylactic acid (PLA), polyglycolic acid (PGA), and a co-polymer comprising polylactic acid and polyglycolic acid (PLGA), the polyester being entangled with hyaluronic acid, for use in a method for repairing a hyaline cartilage defect in a mammalian joint.

2. The membrane for use according to Claim 1 wherein the mammalian joint is a human joint.

3. The membrane for use according to Claims 1 or 2 wherein the hyaline cartilage defect comprises a full-thickness chondral defect.

4. The membrane for use according to any one of Claims 1 to 3 wherein the membrane has a thickness of at least 0.5mm up to about 3mm.

5. The membrane for use according to any one of Claims 1 to 4 wherein the polyester is a co-polymer comprising polylactic acid and polyglycolic acid (PLGA).

6. The membrane for use according to Claim 5 wherein the polylactic acid and the polyglycolic acid are in a weight ratio of from about 5:1 to about 2:1.

7. The membrane for use according to Claim 6, wherein the polylactic acid and the polyglycolic acid are in a weight ratio of about 3:1.

8. The membrane for use according to any one of Claims 1 to 7 wherein the mammalian joint is a joint comprising articular cartilage.

9. The membrane for use according to any one of Claims 1 to 8 wherein the polyester and the hyaluronic acid are in a weight ratio of from 99:1 to 1:99.

10. The membrane for use according to any one of Claims 1 to 8 wherein the polyester and the hyaluronic acid are in a weight ratio of from about 9:1 to 1:9.

11. The membrane for use according to any one of Claims 1 to 10 wherein the membrane further comprises at least one growth factor selected from the group consisting of a TG93, a bone morphogenetic protein, a growth differentiation factor, ADMP-1, a fibroblast growth factor, a hedgehog protein, an insulin-like growth factor, a platelet-derived growth factor, an interleukin, a colony-stimulating factor, and an activin.

12. The membrane for use according to any one of Claims 1 to 11 wherein the membrane further comprises at least one collagen.

## Patentansprüche

1. Membran in Kombination mit autologen mesenchymalen Stammzellen, wobei die Membran ein Polyester umfasst, ausgewählt aus der Gruppe bestehend aus Polymilchsäure (PLA), Polyglykolsäure (PGA) und ein Copolymer, das Polymilchsäure und Polyglykolsäure umfasst (PLGA), wobei das Polyester mit Hyaluronsäure verworren ist, zur Verwendung in einem Verfahren zum Reparieren eines hyalinen Knorpeldefekts in einem Säugetiergelenk.

2. Membran zur Verwendung nach Anspruch 1, wobei das Säugetiergelenk ein menschliches Gelenk ist.

3. Membran zur Verwendung nach Anspruch 1 oder 2, wobei der hyaline Knorpeldefekt einen chondralen Vollschichtdefekt umfasst.

4. Membran zur Verwendung nach einem der Ansprüche 1 bis 3, wobei die Membran eine Dicke von wenigstens 0,5 mm bis ungefähr 3 mm aufweist.

5. Membran zur Verwendung nach einem der Ansprüche 1 bis 4, wobei das Polyester ein Copolymer ist, das Polymilchsäure und Polyglykolsäure umfasst (PLGA).

6. Membran zur Verwendung nach Anspruch 5, wobei die Polymilchsäure und die Polyglykolsäure ein Gewichtsverhältnis von ungefähr 5:1 bis ungefähr 2:1 aufweisen.

7. Membran zur Verwendung nach Anspruch 6, wobei die Polymilchsäure und die Polyglykolsäure ein Gewichtsverhältnis von ungefähr 3:1 aufweisen.

8. Membran zur Verwendung nach einem der Ansprüche 1 bis 7, wobei das Säugetiergelenk ein Gelenk ist, das artikularen Knorpel umfasst.

9. Membran zur Verwendung nach einem der Ansprüche 1 bis 8, wobei das Polyester und die Hyaluronsäure ein Gewichtsverhältnis von 99:1 bis 1:99 aufweisen.

10. Membran zur Verwendung nach einem der Ansprüche 1 bis 8, wobei das Polyester und die Hyaluronsäure ein Gewichtsverhältnis von ungefähr 9:1 bis 1:9 aufweisen.

11. Membran zur Verwendung nach einem der Ansprüche 1 bis 10, wobei die Membran ferner wenigstens einen Wachstumsfaktor umfasst, ausgewählt aus der Gruppe bestehend aus einem TG93, einem knochenmorphogenetischen Protein, einem Wachstumsdifferenzierungsfaktor, ADMP-1, einem Fibroblasten-Wachstumsfaktor, einem Hedgehog-Protein, einem insulinähnlichen Wachstumsfaktor, einem von Thrombozyten abgeleiteten Wachstumsfaktor, einem Interleukin, einem koloniesimulierenden Faktor und einem Aktivin.

12. Membran zur Verwendung nach einem der Ansprüche 1 bis 11, wobei die Membran ferner wenigstens ein Kollagen umfasst.

## Revendications

1. Membrane combinée à des cellules souches mésenchymateuse autologues, ladite membrane comprenant un polyester choisi dans l'ensemble constitué de l'acide polylactique (PLA), de l'acide polyglycolique (PGA) et d'un copolymère comprenant l'acide polylactique et l'acide polyglycolique (PLGA), le polyester étant enchevêtré avec de l'acide hyaluronique, utilisé dans un procédé de réparation d'un défaut du cartilage hyalin dans une jointure de mammifère.

2. Membrane à employer selon la revendication 1, l'articulation de mammifère étant une articulation humaine.

3. Membrane à employer selon la revendication 1 ou 2, le défaut de cartilage hyalin comprenant un défaut chondral d'épaisseur complète.

4. Membrane à employer selon l'une quelconque des revendications 1 à 3, la membrane ayant une épaisseur d'au moins 0,5 mm et pouvant atteindre environ 3 mm.

5. Membrane à employer selon l'une quelconque des revendications 1 à 4, le polyester étant un copolymère contenant de l'acide polylactique et de l'acide polyglycolique (PLGA).

6. Membrane à employer selon la revendication 5, dans laquelle l'acide polylactique et l'acide polyglycolique sont présents selon un rapport pondéral d'environ 5:1 à environ 2:1.

7. Membrane à employer selon la revendication 6, dans laquelle l'acide polylactique et l'acide polyglycolique sont présents selon un rapport pondéral d'environ 3:1.

8. Membrane à employer selon l'une quelconque des revendications 1 à 7, 'l'articulation de mammifère étant une articulation comprenant un cartilage articulaire.

9. Membrane à employer selon l'une quelconque des revendications 1 à 8, dans laquelle le polyester et l'acide hyaluronique sont présents selon un rapport pondéral d'environ 99:1 à environ 1:99.

10. Membrane à employer selon l'une quelconque des revendications 1 à 8, dans laquelle le polyester et l'acide hyaluronique sont présents selon un rapport pondéral d'environ 9:1 à environ 1:9.

11. Membrane à employer selon l'une quelconque des revendications 1 à 10, la membrane comprenant en outre au moins un facteur de croissance choisi dans l'ensemble constitué d'un TG93, d'une protéine morphogénétique osseuse, d'un facteur de différentiation de croissance, d'ADMP-1, d'un facteur de croissance de fibroblaste, d'une protéine de hérisson, d'un facteur de croissance de type insuline, d'un facteur de croissance dérivé d'une plaquette, d'une interleucine, d'un facteur de stimulation de colonie et d'une activine.

12. Membrane à employer selon l'une quelconque des revendications 1 à 11, la membrane comprenant en outre au moins un collagène.
